# EUROPÄISCHE PATENTSCHRIFT

(11) **EP 2 456 397 B1**
(45) Veröffentlichungstag und Bekanntmachung des Hinweises auf die Patenterteilung: **12.06.2013**
(21) Anmeldenummer: 10736622.1
(22) Anmeldetag: 19.07.2010
(51) Int. Cl.: A61F 2/50, A61F 2/68, B60W 50/08, G06F 3/01

(54) **VORRICHTUNG UND EIN VERFAHREN ZUR ERZEUGUNG EINES VIBRATIONSMUSTERS**
DEVICE AND METHOD FOR GENERATING A VIBRATION PATTERN
DISPOSITIF ET PROCÉDÉ DE PRODUCTION D UN MODÈLE DE VIBRATION

(30) Priorität: 24.07.2009 DE 102009034708
(43) Veröffentlichungstag der Anmeldung: 30.05.2012
(73) Patentinhaber: Otto Bock Healthcare Products GmbH, 1070 Wien (AT)
(72) Erfinder: NINU, Andrei, A-1140 Wien (AT)
(74) Vertreter: Stornebel, Kai
(86) Internationale Anmeldenummer: PCT/EP2010/004386
(87) Internationale Veröffentlichungsnummer: WO 2011/009576

(56) Entgegenhaltungen:
- US-A- 5 413 611
- US-A1- 2002 052 663
- US-B1- 6 500 210

## Beschreibung

Die Erfindung betrifft eine Vorrichtung und ein Verfahren zur Erzeugung eines Vibrationsmusters bei einer Person mit einem Antrieb, der eine Masse in eine Drehbewegung versetzt, sowie zumindest einer Sensoreinrichtung, die mit einer Steuerung gekoppelt ist, die den Antrieb in Abhängigkeit von Sensordaten der Sensoreinrichtung steuert.

Die US 5,413,611 beschreibt eine Prothese mit einer Feedback-Einrichtung. Über einen Vibrationserzeuger wird eine Rückmeldung über die aufgebrachte Kraft einer angetriebenen Prothese erzeugt.

Die US 2002/0052663 A1 betrifft eine Protheseneinrichtung mit einem Prothesenkniegelenk und einem Vibrationsgenerator zur Erzeugung eines Warnsignals. Der Vibrationsgenerator kann als Taumelmotor oder Wubbelmotor ausgebildet sein.

Die US 2004/0178989 A1 beschreibt ein System und ein Verfahren zum Bereitstellen einer haptischen Rückkopplung, bei der ein Motor mit einer exzentrisch angeordneten Masse in Bewegung versetzt wird, um ein Vibrationsmuster zu erzeugen. Eine ähnliche Einrichtung ist in der WO 03/091984 A1 beschrieben.

Aufgabe der vorliegenden Erfindung ist es, eine Vorrichtung zur Erzeugung eines Vibrationsmusters und ein Verfahren bereitzustellen, bei dem komplexe Feedbackmuster leicht erzeugt werden können. Erfindungsgemäß wird dies durch eine Vorrichtung mit den Merkmalen des Hauptanspruches und durch ein Verfahren mit den Merkmalen des nebengeordneten Anspruchs gelöst. Vorteilhafte Ausgestaltungen und Weiterbildungen der Erfindung sind in den Unteransprüchen aufgeführt.

Die erfindungsgemäße Vorrichtung zur Erzeugung eines Vibrationsmuster bei einer Person mit einem Antrieb, der eine Masse in eine Drehbewegung versetzt, sowie zumindest einer Sensoreinrichtung, die mit einer Steuerung gekoppelt ist, die den Antrieb in Abhängigkeit von Sensordaten der Sensoreinrichtung steuert, sieht vor, dass die Masse konzentrisch homogen um ihre Drehachse herum angeordnet ist und dass die Vorrichtung eine Schnittstelle aufweist, die Reaktionskräfte der Masse, die aufgrund der Veränderung der Drehbewegung entstehen, auf den Patienten überträgt. Die Vorrichtung weist eine homogene konzentrische Masse auf, die von einem Antrieb in eine Drehbewegung versetzt wird. Die Erzeugung des Vibrationsmusters basiert dabei auf dem Prinzip der Aktion-Reaktion. Durch das Beschleunigen der rotierenden Masse entsteht eine gerichtete Trägheitskraft, die einen Stator, um den die konzentrische Masse dreht, zu einer Gegenbewegung veranlasst. Die dabei entstehende Reaktionskraft erzeugt ein für den Benutzer spürbares Feedback.

Vorzugsweise ist die rotierende Masse als ein Rotor ausgebildet, der um einen Stator des Antriebes herum gelagert ist, sodass die Vorrichtung sehr kleinbauend ausgebildet werden kann.

Die Sensoreinrichtung, die die Daten liefert, auf deren Grundlage die Steuereinheit errechnet, wie groß der überstrichene Rotorwinkel, die Winkelgeschwindigkeit und die Beschleunigung sein müssen, können als Drucksensor, Lagesensor, Momentsensor, Bewegungssensor und/oder Temperatursensor ausgebildet sein. Dadurch ist es möglich, eine Vielzahl an Einflüssen aufzunehmen und mittels eines Vibrationsmusters an den Nutzer der Vorrichtung zu übertragen.

Beispielsweise kann die Sensoreinrichtung ebenso wie die Vibrationseinrichtung in oder an einer Exoprothese angeordnet oder mit einer Exoporthese gekoppelt sein. So ist es beispielsweise möglich, Zustände der Prothesenelemente innerhalb der Exoprothese zu verarbeiten und über die Vorrichtung auf den Stumpf oder die Befestigungsstelle der Prothese an den Körper zu übertragen. Alternative Anbringungsorte der Vorrichtung stehen ebenfalls zur Verfügung, grundsätzlich sind alle ausreichend empfindlichen Körperstellen dazu geeignet.

An der Vorrichtung kann eine Befestigungsanordnung zum Anbringen der Vorrichtung an der Person angeordnet sein. Die Befestigungsanordnung kann als Gurt, Manschette oder Spange ausgebildet sein. Ebenfalls ist es möglich, dass über ein Kopplungsmittel das Vibrationsmuster auf den Körper übertragen wird. Das Kopplungsmittel kann die Vibration konzentrieren, beispielsweise indem der Stator über einen Ausleger oder eine Einrichtung mit einer Auflagefläche, die kleiner als der Stator ist, mit dem Nutzer der Vorrichtung gekoppelt wird. Das Kopplungselement, das die Reaktionskräfte der Masse auf die Person überträgt, kann abnehmbar an der Vorrichtung angeordnet oder ein integraler Bestandteil der Vorrichtung sein. Sofern das Kopplungselement über einen Hebel mit einem Stator des Antriebes verbunden ist, kann eine Vibrationsübertragung angepasst erfolgen. Ebenfalls ist es möglich, durch eine Hebelanordnung eine Getriebeübersetzung vorzunehmen, über die eine Amplitudenverstärkung durchgeführt wird.

Das erfindungsgemäße Verfahren zur Erzeugung eines Feedback-Signals, bei dem über zumindest eine Sensoreinrichtung ein Sensorsignal bereitgestellt wird und eine konzentrisch und homogen um eine in Drehachse angeordnete Masse in Abhängigkeit von dem Sensorsignal angetrieben wird, sieht vor, dass ein frequenz- und/oder amplitudenmoduliertes Feedbackmuster erzeugt wird. Die Masse wird dabei in unterschiedlichen Drehrichtungen und mit unterschiedlichen Winkelgeschwindigkeiten angetrieben, je nach Sensorsignal und korrespondierend dazu zu erzeugendem Feedbackmuster. Die Masse zur Erzeugung des Feedbackmusters wird in Abhängigkeit von dem Sensorsignal in unterschiedlichen Beschleunigungsmustern angetrieben, wobei die Beschleunigungsmuster sowohl in einer Frequenzmodulation als auch in einer Amplitudenmodulation variiert werden können. Die Amplitudenmodulation wird durch die Beschleunigung des Rotors geregelt. Die Regelung der Beschleunigung wird aufgrund gemessener Bewegungsgrößen des Rotors durchgeführt, je stärker die Beschleunigung des Rotors ist, desto höher ist die Amplitude der Stimulation. Die Frequenz ist eine Funktion der Winkelgeschwindigkeit und des überstrichenen Rotorwinkels. Durch Synchronisation der Winkelgeschwindigkeit mit den überstrichenen Rotorwinkeln ist es möglich, die Frequenz der Stimulation in der gewünschten Weise zu modulieren. Dabei kann die Frequenz konstant oder variabel sein.

Es ist möglich, dass das Signal zeitabhängig oder in Abhängigkeit von einem Zustand der Vorrichtung oder deren Anbauteile erzeugt wird. Die Vorrichtung kann durch bestimmte Zustände, beispielsweise der Prothese, angetriggert werden. Beispielsweise kann durch eine automatische Umschaltung der Prothese in einen anderen Modus die Information über ein anderes Prothesenverhalten durch ein entsprechendes Signal an den Prothesenträger weitergegeben werden. Ebenfalls ist es möglich, bestimmte Zustände, beispielsweise einen Ladezustand eines Akkumulators, einen eingeschalteten Prothesenzustand oder eine Überhitzung von Prothesenkomponenten, durch ein entsprechendes Vibrationsmuster anzuzeigen.

Grundsätzlich ist es auch möglich, die Vorrichtung mit einer Prothese über ein Kabel oder über eine Funkstrecke zu verbinden. Dadurch ist es möglich, Zustände, die innerhalb einer Prothese ermittelt werden, an einer entfernten Stelle über ein Vibrationsmuster zu übermitteln. Dabei ist die Anwendung der Vorrichtung nicht auf die Übermittlung von Daten betreffend eine Prothese beschränkt. Ebenfalls ist es möglich, ein Feedback von Trainingsgeräten zu erhalten oder aber auch ein Feedback von myoelektrischen Signalen, die von entsprechenden Ableiterelektroden erfasst wurden.

Nachfolgend werden Ausführungsbeispiele der Erfindung anhand der beigefügten Figuren näher erläutert. Es zeigen:
- Figur 1 -: eine Darstellung der Funktionsweise der Vorrichtung;
- Figur 2 -: eine perspektivische Ansicht eines Vibrationserzeugers;
- Figur 3 -: eine Schnittansicht;
- Figur 4 -: eine schematische Darstellung einer Vorrichtung;
- Figur 5 -: eine Darstellung einer angelegten Vorrichtung;
- Figur 6 -: unterschiedliche Anordnungen an einer Person; sowie
- Figur 7 -: Darstellungen von Frequenz- und Amplitudenmodulationen.

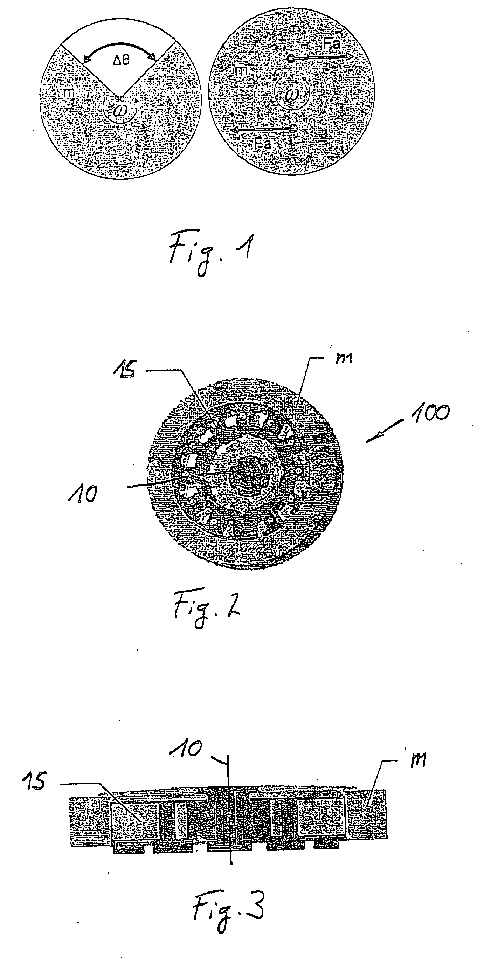

In der Figur 1 ist der prinzipielle Aufbau einer Vorrichtung zur Erzeugung eines Vibrationsmusters gemäß der vorliegenden Erfindung dargestellt. Die Vorrichtung weist eine homogene, konzentrische Masse m auf, die von einem elektrischen Antrieb, der nicht dargestellt ist, mit einem überstrichenen Rotorwinkel Δθ, der Winkelgeschwindigkeit ω und der Beschleunigung a = dω/dt gedreht wird. Der überstrichene Rotorwinkel Δθ bzw. die Winkelgeschwindigkeit ω werden entweder über Sensoren unmittelbar gemessen oder indirekt durch eine Umrechnung der Rotorbewegungsgrößen ermittelt. Die ermittelte Rotorposition bzw. die momentane Winkelgeschwindigkeit werden von der nicht dargestellten Motorsteuereinheit genutzt, um vorgegebene Vibrationsmuster auf der Grundlage von Sensordaten, die von einer Sensoreinrichtung bereitgestellt werden, die nicht die Rotorbewegungen ermittelt, zu erzeugen. Es handelt sich also um eine Einrichtung zur Übermittlung eines Feedbacks über Vibrationsmuster, wobei die Erzeugung des Feedbacks auf dem Prinzip der Aktion-Reaktion basiert. Durch das Beschleunigen der rotierenden Masse m entsteht eine gerichtete Trägheitskraft Fa gemäß der Formel Fa = m * a. Diese gerichtete Trägheitskraft Fa veranlasst den Stator 15 des Motors zu einer Gegenbewegung. Die dabei entstehende Reaktionskraft erzeugt ein für den Benutzer spürbaren Impuls und damit ein Feedback.

In der Figur 2 ist in einer perspektivischen Darstellung einer Ausführungsform die rotierende Masse m in Gestalt eines Ringes gegeben, der konzentrisch und homogen um die Drehachse 10 herum angeordnet ist. Die Drehachse 10 liegt im Zentrum eines Stators 15, der als Stator eines Motors ausgebildet ist. Die Masse m ist also Teil des Antriebes, nämlich Teil eines Elektromotors. Neben der dargestellten integrierten Lösung, bei der die Masse m nicht separat gelagert ist, besteht die Möglichkeit, dass der Antrieb über eine Getriebe oder eine Übersetzung mit der drehbar gelagerten Masse m gekoppelt ist. Um den Stator 15 herum ist der bewegliche Rotor als rotierende Masse m angeordnet, sodass sich hier die Bauform des Außenläufer-Elektromotors ergibt. Die rotierende Masse m kann bei der Ausgestaltung als elektronisch kommutierter Gleichstrommotor aus ringförmig angeordneten Dauermagneten bestehen oder diese aufweisen, um die Drehbewegung der rotierenden Masse m zu erzeugen. Durch eine entsprechende Ansteuerung der Erregerspulen innerhalb des Stators 15 ist es möglich, ein Vibrationsmuster als Feedbackmuster amplituden- und/oder frequenzmoduliert zu erzeugen. Zusätzlich ermöglicht es die Überlagerung von mehreren modulierten Feedbackmustern und dadurch die Erzeugung einer großen Variation hochkomplexer Feedbackmuster, sodass eine Vielzahl von Sensordaten unterschiedlichster Art über unterschiedlichste Feedbackmuster einfach und zuverlässig an den Nutzer der Vorrichtung 100 weitergegeben werden können.

Die Amplitudenmodulation wird durch die Beschleunigung des Rotors m geregelt. Befindet sich die rotierende Masse m bereits in einer Drehbewegung, wird der gegenwärtige Zustand durch die Motorsteuerung erfasst. Auf der Grundlage der gemessenen oder ermittelten Bewegungsgrößen der rotierenden Masse m wird dann die Regelung der Beschleunigung durchgeführt, sodass aufgrund der Veränderung der Winkelgeschwindigkeit die gerichtete Trägheitskraft Fa erzeugt wird, die sich über den Stator 15 auf den Nutzer der Vorrichtung 100 übertragen lässt. Je stärker die Beschleunigung der rotierenden Masse m, also sowohl im Sinne einer positiven als auch einer negativen Beschleunigung, desto höher ist die Amplitude der Stimulation und die Vibrationsamplitude. Steht die Masse m anfänglich still, wird der entsprechende Vibrationseffekt durch Beschleunigen in die eine und/oder andere Richtung erreicht.

Die Frequenz der erzeugten Vibration ist eine Funktion der Winkelgeschwindigkeit ω und des überstrichenen Rotorwinkels Δθ. Durch Synchronisation der Winkelgeschwindigkeit ω mit den überstrichenen Rotorwinkeln Δθ ist es möglich, die Frequenz der Stimulation in der gewünschten Weise zu modulieren. Die Frequenz kann dabei entweder konstant oder variabel sein. Die Frequenz der Stimulation ergibt sich aus dem Quotienten der Winkelgeschwindigkeit ω zu dem überstrichenen Rotorwinkel Δθ.

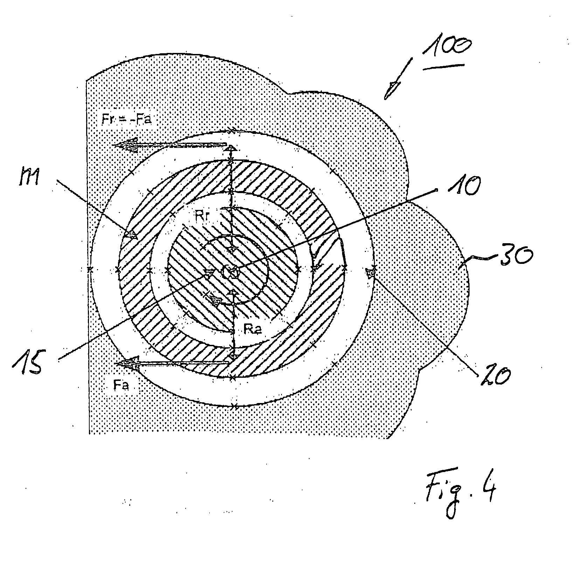

In der Figur 4 ist die Vorrichtung 100 vergrößert und schematisch gezeigt. Die Vorrichtung 100 ist dabei auf der Hautoberfläche 30 eines Nutzers angeordnet. Die rotierende Masse m dreht sich um die Drehachse 10, um die herum der Stator 15 angeordnet ist. Die Vorrichtung 100 sieht im dargestellten Ausführungsbeispiel vor, dass die Masse m Teil des Antriebs ist, nämlich des Elektromotors aus Stator 15 und die Masse m ausbildenden Rotor. Der Stator 15 des Motors generiert ein magnetisches Drehfeld, aufgrund dessen die rotierende Masse m in Abhängigkeit von der Art des Drehfeldes gedreht wird. Der Stator 15 ist dabei an einem Gehäuse 20 festgelegt, das die Schnittstelle zwischen der Vorrichtung 100 und der Hautoberfläche 30 bildet. Je nach Drehrichtung, Frequenz und Amplitude der Änderung wird ein entsprechendes Muster an Reaktionskräften und damit an Vibrationen erzeugt.

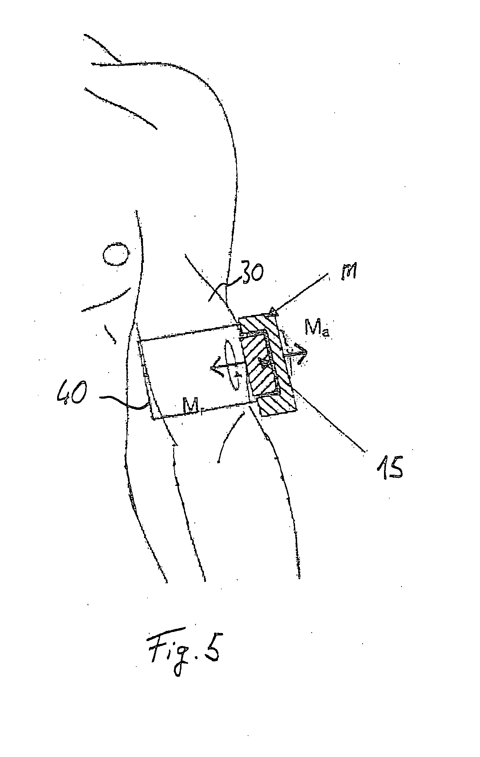

In der Figur 5 ist dabei die Vorrichtung 100 im angelegten Zustand gezeigt. In der Figur 5 ist die Vorrichtung 100 an einem Oberarm über eine Befestigungsanordnung 40 in Gestalt einer Manschette angelegt. In der Figur 5 sind auch die wirkenden Momente gezeigt. Das Reaktionsmoment Mr wird von der rotierenden Masse m auf die Schnittstelle, also auf das Gehäuse 20 ausgeübt. Die Größe des Momentes Mr wird durch das Trägheitsmoment der rotierenden, homogenen konzentrischen Masse m und der darauf einwirkenden Kraft bzw. Beschleunigung definiert.

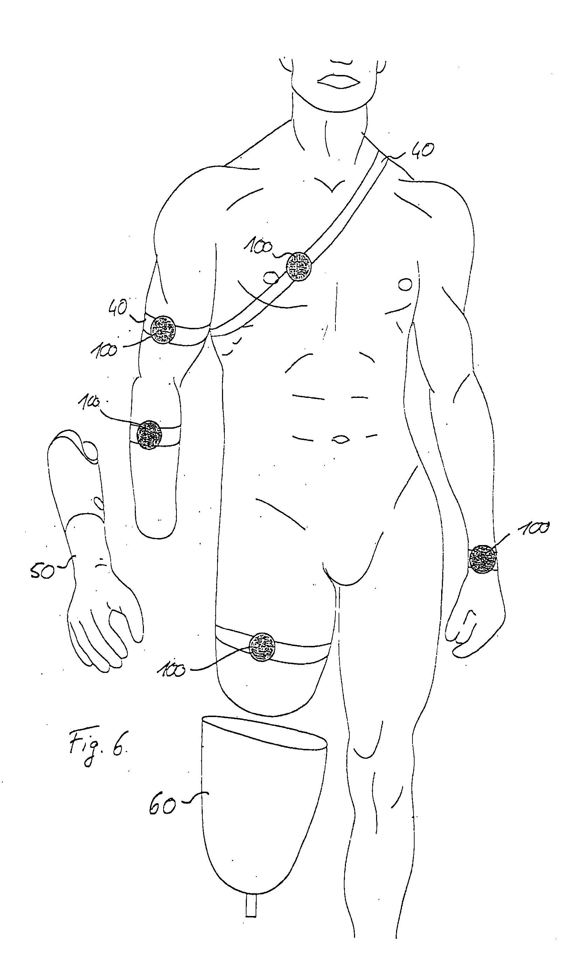

In der Figur 6 sind unterschiedliche Anbringungsstellen der Vorrichtung 100 gezeigt, beispielsweise am Handgelenk, am Brustkorb, am Oberarm, am Unterarm oder am Oberschenkel. Ebenfalls ist es möglich, die Vorrichtung unmittelbar in einer Protheseneinrichtung 50, 60 anzuordnen, beispielsweise in einer Unterarmprothese 50 oder in einem Oberschenkelschaft 60 einer Beinprothese.

In der Figur 7 sind eine Frequenzmodulation und eine Amplitudenmodulation dargestellt. Bei einer sprungweise, in ihrem Betrag gleichbleibenden Beschleunigung a verändert sich die Stärke des Vibrationsimpulses nicht, da über den Verlauf der Zeit das Beschleunigen bzw. Abbremsen oder die Bewegungsumkehr in immer kürzeren Zeitabständen erfolgt, erhöht sich die Vibrationsfrequenz, sodass der Nutzer der Vorrichtung ein Feedback erhält, das in Abhängigkeit von den gemessenen Sensorsignalen erzeugt wird. Die positive und negative Beschleunigung a bedeutet, dass entweder die Drehrichtung umgekehrt oder die Masse m um eine Grundgeschwindigkeit herum beschleunigt und abgebremst wird.

In der rechten Darstellung der Figur 7 ist hingegen eine Amplitudenmodulation gezeigt, bei der das Maß der Beschleunigung a variiert wird, ohne die Frequenz zu verändern. In dem dargestellten Ausführungsbeispiel führt dies dazu, dass zunächst eine schwache Vibration auf den Nutzer übertragen wird, die mit zunehmenden Zeitablauf vergrößert wird, bis sie dann nach Erreichen des Maximums wieder abschwillt. Durch eine Überlagerung der Frequenzmodulation und der Amplitudenmodulation können vielfältige Signalmuster erzeugt und auf den Nutzer der Vorrichtung übertragen werden.

Neben Daten, beispielsweise zum Zustand einer Protheseneinrichtung 50, 60, beispielsweise der aktuellen Griffkraft, können andere Daten über die Vorrichtung 100 an den Nutzer weitergeleitet werden, beispielsweise Zustandsdaten über Temperatur, Einschaltzustände, Betriebsmodi, Batteriezustände oder dergleichen. Ebenfalls können myoelektrische Daten, die über Ableiterelektroden aufgenommen werden, verarbeitet und als Vibrationssignale an den Nutzer weitergegeben werden, beispielsweise um ein optimiertes Training durchführen zu können.

## Patentansprüche

1. Vorrichtung zur Erzeugung eines Vibrationsmusters bei einer Person mit einem Antrieb, der eine Masse in eine Drehbewegung versetzt, sowie zumindest einer Sensoreinrichtung, die mit einer Steuerung gekoppelt ist, die den Antrieb in Abhängigkeit von Sensordaten der Sensoreinrichtung steuert, **dadurch gekennzeichnet, dass** die Masse (m) konzentrisch homogen um ihre Drehachse (10) herum angeordnet ist und dass eine Schnittstelle (20) Reaktionskräfte der Masse (m), die aufgrund der Veränderung der Drehbewegung entstehen, auf die Person überträgt.

2. Vorrichtung nach Anspruch 1, **dadurch gekennzeichnet, dass** der Antrieb ein Elektromotor ist.

3. Vorrichtung nach Anspruch 1 oder 2, **dadurch gekennzeichnet, dass** die rotierende Masse (m) als Rotor ausgebildet ist, der um einen Stator (15) des Antriebes herum gelagert ist.

4. Vorrichtung nach einem der voranstehenden Ansprüche, **dadurch gekennzeichnet, dass** die Sensoreinrichtung als Drucksensor, Lagesensor, Momentensensor, Bewegungssensor und/oder Temperatursensor ausgebildet ist.

5. Vorrichtung nach einem der voranstehenden Ansprüche, **dadurch gekennzeichnet, dass** die Sensoreinrichtung in einer Exoprothese (50, 60) angeordnet ist.

6. Vorrichtung nach einem der voranstehenden Ansprüche, **dadurch gekennzeichnet, dass** die Vorrichtung (100) in oder an einer Exoprothese (50, 60) angeordnet oder mit der Exoprothese (50, 60) gekoppelt ist.

7. Vorrichtung nach einem der voranstehenden Ansprüche, **dadurch gekennzeichnet, dass** eine Befestigungsanordnung (40) zum Anbringen der Vorrichtung (100) an der Person vorgesehen ist.

8. Vorrichtung nach einem der voranstehenden Ansprüche, **dadurch gekennzeichnet, dass** zumindest ein Kopplungselement vorgesehen ist, das die Reaktionskräfte der Masse (m) auf die Person überträgt.

9. Vorrichtung nach Anspruch 8, **dadurch gekennzeichnet, dass** das Kopplungselement über einen Hebel mit einem Stator (15) des Antriebes verbunden ist.

10. Verfahren zur Erzeugung eines Feedback-Signals, bei dem über zumindest eine Sensoreinrichtung ein Sensorsignal bereitgestellt wird und eine konzentrisch und homogen um eine Drehachse angeordnete Masse (m) in Abhängigkeit von dem Sensorsignal angetrieben wird, um durch Reaktionskräfte der Masse (m) ein frequenz- und/oder amplitudenmoduliertes Feedbackmuster zu erzeugen.

11. Verfahren nach Anspruch 10, **dadurch gekennzeichnet, dass** die Masse in unterschiedlichen Drehrichtungen angetrieben wird.

12. Verfahren nach Anspruch 10 oder 11, **dadurch gekennzeichnet, dass** die Masse zur Erzeugung des Feedback-Signals in Abhängigkeit von dem Sensorsignal in unterschiedlichen Beschleunigungsmustern angetrieben wird.

13. Verfahren nach einem der Ansprüche 10 bis 12, **dadurch gekennzeichnet, dass** mehrere Feedbackmuster einander überlagert werden.

14. Verfahren nach einem der Ansprüche 10 bis 14, **dadurch gekennzeichnet, dass** das Sensorsignal zeitabhängig oder in Abhängigkeit von einem Zustand der Vorrichtung oder deren Anbauteile erzeugt wird.

## Claims

1. A device for generating a vibration pattern in a person, having a drive which sets a mass into rotation and at least one sensor apparatus that is coupled to a control which controls the drive dependent on sensor data from the sensor apparatus, **characterized in that** the mass (m) is arranged in a concentric homogeneous fashion about the rotational axis (10) thereof and **in that** an interface (20) transmits reaction forces to the person from the mass (m), which arise as a result of the change in the rotation.

2. The device as claimed in claim 1, **characterized in that** the drive is an electric motor.

3. The device as claimed in claim 1 or 2, **characterized in that** the rotating mass (m) is embodied as a rotor which is mounted about a stator (15) of the drive.

4. The device as claimed in one of the preceding claims, **characterized in that** the sensor apparatus is embodied as a pressure sensor, position sensor, torque sensor, movement sensor and/or temperature sensor.

5. The device as claimed in one of the preceding claims, **characterized in that** the sensor apparatus is arranged in an exoprosthesis (50, 60).

6. The device as claimed in one of the preceding claims, **characterized in that** the device (100) is arranged in or on an exoprosthesis (50, 60) or coupled to the exoprosthesis (50, 60).

7. The device as claimed in one of the preceding claims, **characterized in that** provision is made for an attachment arrangement (40) for attaching the device (100) to the person.

8. The device as claimed in one of the preceding claims, **characterized in that** provision is made for at least one coupling element which transmits the reaction forces to the person from the mass (m).

9. The device as claimed in claim 8, **characterized in that** the coupling element is connected to a stator (15) of the drive via a lever.

10. A method for generating a feedback signal, in which a sensor signal is provided by at least one sensor apparatus and a mass (m), arranged concentrically and homogeneously about a rotational axis, is driven depending on the sensor signal in order to generate a frequency- and/or amplitude-modulated feedback pattern by the reaction forces of the mass (m).

11. The method as claimed in claim 10, **characterized in that** the mass is driven in different rotational directions.

12. The method as claimed in claim 10 or 11, **characterized in that** the mass for generating the feedback signal is driven with different acceleration patterns depending on the sensor signal.

13. The method as claimed in one of claims 10 to 12, **characterized in that** a plurality of feedback patterns are superposed on one another.

14. The method as claimed in one of claims 10 to 14, **characterized in that** the sensor signal is generated in a time-dependent fashion or dependent on a state of the device or the attachment parts thereof.

## Revendications

1. Dispositif de production d'un modèle de vibration sur une personne, comprenant un entraînement qui met une masse en mouvement de rotation, et comprenant un dispositif capteur couplé à une commande, laquelle pilote l'entraînement en fonction de données de capteur du dispositif capteur, **caractérisé en ce que** la masse (m) est disposée de façon concentrique et homogène autour de son axe de rotation (10), et **en ce qu'**une interface (20) transmet à la personne des forces de réaction de la masse (m) qui apparaissent en raison de la modification du mouvement de rotation.

2. Dispositif selon la revendication 1, **caractérisé en ce que** l'entraînement est un moteur électrique.

3. Dispositif selon la revendication 1 ou 2, **caractérisé en ce que** la masse rotative (m) est agencée en rotor qui est monté autour d'un stator (15) de l'entraînement.

4. Dispositif selon l'une des revendications précédentes, **caractérisé en ce que** le dispositif capteur est réalisé sous forme de capteur de pression, capteur de position, capteur de moment, capteur de mouvement et/ou capteur de température.

5. Dispositif selon l'une des revendications précédentes, **caractérisé en ce que** le dispositif capteur est agencé dans une exoprothèse (50, 60).

6. Dispositif selon l'une des revendications précédentes, **caractérisé en ce que** le dispositif (100) est agencé dans ou sur une exoprothèse (50, 60) ou bien est couplé à l'exoprothèse (50, 60).

7. Dispositif selon l'une des revendications précédentes, **caractérisé en ce qu'**il est prévu un agencement de fixation (40) pour installer le dispositif (100) sur la personne.

8. Dispositif selon l'une des revendications précédentes, **caractérisé en ce qu'**il est prévu au moins un élément de couplage qui transmet les forces de réaction de la masse (m) à la personne.

9. Dispositif selon la revendication 8, **caractérisé en ce que** l'élément de couplage est relié par un levier avec un stator (15) de l'entraînement.

10. Procédé pour produire un signal de contre-réaction, dans lequel on tient à disposition un signal de capteur par l'intermédiaire d'au moins un dispositif capteur et on entraîne en fonction du signal de capteur une masse (m) disposée de façon concentrique et homogène autour d'un axe de rotation, pour produire par des forces de réaction de la masse (m) un modèle de contre-réaction modulé en fréquence et/ou en amplitude.

11. Procédé selon la revendication 10, **caractérisé en ce qu'**on entraîne la masse dans des sens de rotation différents.

12. Procédé selon la revendication 10 ou 11, **caractérisé en ce que** pour produire le signal de contre-réaction en fonction du signal de capteur on entraîne la masse dans différents modèles d'accélération.

13. Procédé selon l'une des revendications 10 à 12, **caractérisé en ce qu'**on superpose les uns aux autres plusieurs modèles de contre-réaction.

14. Procédé selon l'une des revendications 10 à 14, **caractérisé en ce qu'**on produit le signal de capteur en fonction du temps ou en fonction d'un état du dispositif ou de ses pièces annexes.
